(19) Europäisches Patentamt European Patent Office Office européen des brevets

(11) **EP 2 113 298 B1**

(12) **EUROPEAN PATENT SPECIFICATION**

(45) Date of publication and mention
of the grant of the patent:
**24.04.2013 Bulletin 2013/17**

(51) Int Cl.:
**B01D 69/08** (2006.01)　　**B01D 71/68** (2006.01)
**B01D 67/00** (2006.01)

(21) Application number: **08008229.0**

(22) Date of filing: **30.04.2008**

(54) **Hollow fibre membrane for hemodialysis with improved permeability and selectivity**

Hohlfasermembran für die Hämodialyse mit verbesserter Durchlässigkeit und Selektivität

Membrane à fibres creuses pour l'hémodialyse dotée d'une perméabilité et d'une sélectivité améliorées

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**04.11.2009 Bulletin 2009/45**

(73) Proprietor: **Gambro Lundia AB**
**22 643 Lund (SE)**

(72) Inventors:
• **Buck, Reinhold**
**88422 Alleshausen (DE)**
• **Deibert, Jenny**
**78628 Rottweil (DE)**
• **Wochner, Arnd**
**72359 Dotternhausen (DE)**

(74) Representative: **Hornung, Veronika Margot**
**c/o Gambro Dialysatoren GmbH**
**Holger-Crafoord-Strasse 26**
**72379 Hechingen (DE)**

(56) References cited:
**EP-A- 0 927 572　　WO-A-2006/106133**
**WO-A-2008/003608　　US-B1- 6 355 730**

Remarks:
The file contains technical information submitted after the application was filed and not included in this specification

EP 2 113 298 B1

**Description**

Technical Field

The present invention relates to a semipermeable asymmetric hollow fiber membrane based on polyethersulfone or polysulfone and PVP, wherein the membrane has a significantly improved permeability and selectivity, to a process for manufacturing such a membrane and to the use of the membrane in, for example, haemodialysis, haemofiltration or haemodiafiltration. Membranes of the above kind present special advantages when they are used in connection with different kinds of medical treatments. They may, however, also be used in dialysis and filtration in general, for example in water purification or dehydration.

Background

Membranes of the above kind are described in detail in, for example, EP 0 568 045 A1, EP 0 168 783 A1, EP 0 082 433 A2, WO 2004/056459 A1, EP 0 750 936 A1 and WO 86/00028 A1. These membranes are manufactured from polymeric synthetic materials; they have an asymmetric structure with high diffusive permeability (clearance) and have water filtration capability in ultrafiltration applications in the range of low flux to high flux. In EP-A-0 305 787, a 3-layer structure membrane and filter with corresponding performance, is disclosed. In Wienk et al. (1998), "A new spinning technique for hollow fiber ultrafiltration membranes", Journal of Membrane Science, 78, 93-100, the use of a high and low molecular weight PVP in a membrane based on polyethersulfone is described for the first time. EP 0 750 936 A1 describes the preparation and use of a polysulfone and PVP based membrane, wherein the PVP consists of a low and a high molecular weight component. WO 2004/056459 A1 describes a 4-layer membrane based on polyethersulfone, PVP and polyamide. Said membrane is characterized by a pore size and density comparable to the one described for the present invention. However, even though this membrane is highly efficient, it does not yet reach to a full extend the exceptional selectivity and permeability of the present membrane, both of which is a function of the membrane geometry but also of membrane composition and spinning technique which is differing from the membrane as described in the present invention.

WO 2006/106133 A1 is directed to an ultrafiltration membrane capable of retaining endotoxins and CIS including bacterial DNA and/or DNA fragments, useful in the filtration of water. The ultrafiltration membrane consists of a polymer blend comprising at least one hydrophobic polymer containing sulfur in its backbone; at least one hydrophilic, uncharges homopolymer of polyvinypyrrolidone; and at least one polymer containing cationic charges.

US 6 355 730 B1 discloses a permselective membrane for hemodialysis having a high permeability and selectivity for uremic toxins. The membrane comprises a polysulfone and a polyvinylpyrrolidone consisting of 10-50 weight-% of a low molecular weight component having a molecular weight of below 100 kD and 90-50 weight-% of a high molecular weight component having a molecular weight of 100 kD or more.

WO 2008/003608 is directed to a plasma separation membrane for application in plasma separation, plasma filtration, micro filtration, plasma therapy or cell filtration applications. The membrane has a sieving coefficient for all plasma proteins of more than 90%.

The membranes according to prior art are well performing, but still have some space for improvement and optimization. For an optimal treatment high values for diffusive permeability (clearance) are desirable. To achieve such values, present day dialysers are designed to have a comparatively big size, both in terms of blood volume and also in terms of membrane surface per dialyser unit. The drawback of such dialysers apparently lies in the high extracorporeal blood volume and the high surface area which is in contact with blood, which increases the risk of adverse reactions of the patient in need of treatment.

It is therefore clearly desirable to devise dialysers which achieve high clearance rates by making use of membranes which have an improved diffusive permeability for substances with low molecular weight (< 500 Dalton), such as urea. Such membranes will allow for at least the same clearance rates as present day dialysers but with a smaller membrane surface area and, as a consequence, smaller dialysers. It is a further advantage of dialyzers with reduced size, that the consumption of raw materials and energy for preparing and shipping them is reduced. Thus, dialyzers with a reduced size and an equally good or improved performance are, in addition, preferable economically and ecologically.

In addition, it is desirable to provide membranes and dialyzers which are able to remove so-called middle sized molecules with high efficiency, while the loss of valuable proteins is low. This is generally referred to as the "selectivity" of a membrane. A high selectivity of a membrane, in the context of the present invention, allows achieving high removal rates for small and middle molecules while minimizing or avoiding the total loss of protein.

Today membranes and dialyzers generally provide for a good removal of such middle molecules at the price of a higher protein loss, or provide for a reduced protein loss at the price of a limited removal of middle sized molecules.

Clinical signs and symptoms of end-stage renal disease patients are at least in part related to the accumulation of "middle molecules" such as, for example, ß2-microglobulin (~12 kD), parathyroid hormone (~7 kD), advanced glycation end products, advanced lipoxidation end products, advanced oxidation protein products (formed as a result of oxidative stress, carbonyl stress, or both), granulocyte inhibitory proteins, or leptin (~16 kD). Currently available membrane materials do not provide a long-lasting, effective reduction of middle molecules in conjunction with a low loss of higher molecular weight protein (above 50 - 60 kD).

The expression "protein" is intended to mean especially albumin and immunoglobins, but also proteins such as orosomucoid, opsonines and other proteins as well as other substances with a molecular weight which is higher than the permeability characteristic of the membrane.

It is therefore desirable to devise a membrane which has a structure and dimensions such that it prevents or minimizes passage of molecules and particles larger than a minimum diameter, which is normally defined in terms of molecular weight for the molecules which cannot pass through the membrane. At the same time, the membrane should allow for the efficient removal of middle molecules. In other words, it is desirable to provide a membrane with improved selectivity. Preferably, such new membrane should have both an improved selectivity and should also have an improved diffusive permeability in comparison with present day membranes and dialysers using such membranes.

The present invention is, therefore, especially directed to a semipermeable membrane with improved selectivity and improved diffusive permeability.

Summary of the Invention

The object of the present invention is to provide improved hollow fiber membranes with regard to diffusive permeability and selectivity, comprised of at least one hydrophobic polymer, such as polyethersulfone or polysulfone, and at least one hydrophilic polymer, being suitable for, for example, haemodialysis.

This object is achieved by a hollow membrane preferably based on the hydrophobic polymer polyethersulfone and on the hydrophilic polymer polyvinylpyrrolidone, wherein the polyvinylpyrrolidone consists of a low molecular weight component having a molecular weight of below 100 kD and a high molecular weight component having a molecular weight of 100 kD or more.

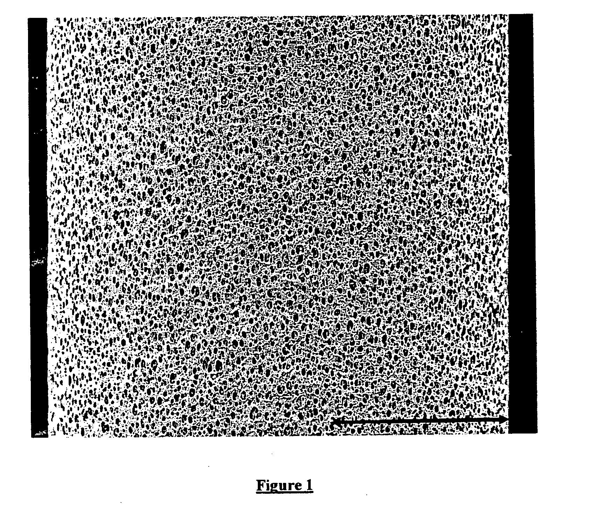

The membrane is characterized by an outer surface having pores in the size range of 0.5 to 3 $\mu$m and having a number of said pores in the range of 10000 to 150000 pores per mm$^2$, preferably in the range of 20000 to 80000 pores per mm$^2$, most preferably 35000 to 55000 pores per mm$^2$ (see Figure 1).

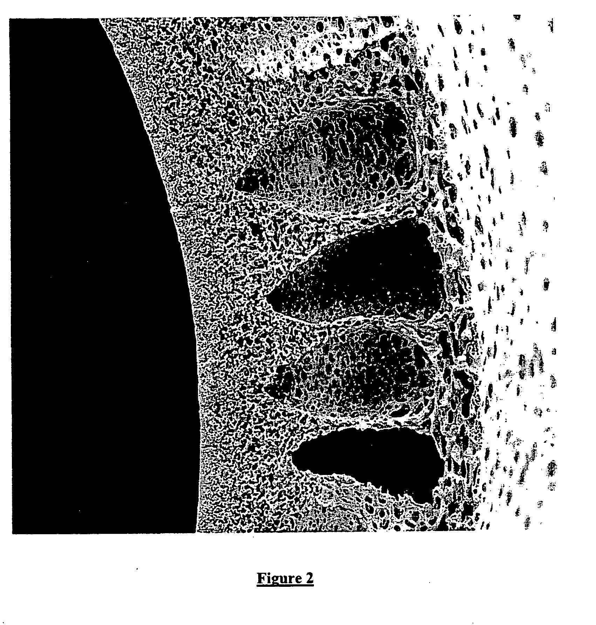

The membrane is further characterized by a very specific four-layer structure and by having a diffusive permeability of chloride of about 19.1·10$^{-4}$ cm/sec measured at 37°C. The diffusive permeability can be determined according to E. Klein, F. Holland, A. Lebeouf, A. Donnaud, J. K. Smith, "Transport and Mechanical Properties of Hemodialysis Hollow Fibers", Journal of Membrane Science 1 (1976) 371-396, especially pages 375-379. In Figure 2, a scanning micrograph is shown for this four-layer structure.

The inner layer of the four-layer structure, i.e. the blood contacting layer and the inner surface of the hollow fiber membrane, is a separation layer in the form of a dense, rather thin layer having, in a preferred embodiment, a thickness of less than 1$\mu$m and a pore size in the nano-scale range. To achieve high selectivity the pore channels with the responsible pore diameters are short, i.e. below 0.1 $\mu$m. The pore channel diameter has a low variation in size. The defined pore structure is achieved by selection of the composition of the polymer, the composition and condition of the precipitation media in the center fluid and by the condition and composition of the surrounding environment of the fiber leaving the spinning nozzle.

The next layer in the hollow fiber membrane is the second layer having the form of a sponge structure and, in a preferred embodiment of the present invention, a thickness of about 1 to 15 $\mu$m and serving as a support for said first layer.

Then, there is the third layer having the form of a finger structure. It provides for mechanical stability on the one hand; on the other hand it has, through the high void volume, a very low resistance of transport of molecules through the membrane. During the process the voids are filled with water and the water gives a lower resistance for diffusion and convection than a matrix with a sponge-filled structure having a lower void volume. Accordingly, the third layer gives the membrane mechanical stability and has, in a preferred embodiment of the present invention, a thickness of 20 to 60 $\mu$m.

The fourth layer is the outer layer, with the outer surface according to above. This fourth layer has in a preferred embodiment a thickness of about 1 to 10 $\mu$m.

This four-layer design provides for a high selectivity, which means, a high potential to separate molecules, which are close in their size, for example, to separate albumin, which is to be retained, from a ß2-microglobulin and Factor D.

The membrane according to the present invention consists of 80-99% by weight of said at least one hydrophobic polymer, preferably polyethersulfone, and 1-20% by weight of said at least one hydrophilic polymer, preferably polyvinylpyrrolidone (PVP). The PVP consists of a high ($\geq$ 100 kD) and low (< 100 kD) molecular component, wherein the PVP consists of 10-45 weight-% based on the total weight of PVP in the membrane, of a high molecular weight component, and of 55-90 weight-% based on the total weight of PVP in the membrane, of a low molecular weight component.

The membrane according to the present invention, due to its specific preparation and membrane characteristics as described before, is especially characterized by a high convective permeability Lp and a high diffusive permeability for small molecules, such as, for example, for urea or chloride ($P_{Cl}$). The Lp is in the range of between $56 \cdot 10^{-4}$ and $84 \cdot 10^{-4}$ cm/bar·s, preferably between 70 and $80 \cdot 10^{-4}$ cm/bar.s. The chloride permeability $P_{Cl}$ is in the range of between $18 \cdot 10^{-4}$ and $21 \cdot 10^{-4}$ cm/s, preferably between $19 \cdot 10^{-4}$ and $20 \cdot 10^{-4}$ cm/s.

The membrane is further characterized by a high selectivity, i.e. a high removal rate for middle molecules while at the same time the loss of protein of higher molecular weight is minimized. The membrane according to the invention has a sieving coefficient ($SC_{Myo}$) for myoglobin (17053 Dalton) in aqueous solution of between 85 and 90%, and a sieving coefficient ($SC_{Albu}$) for albumin (66248 Dalton) in aqueous solution of 9% or less. The selectivity in aqueous solution of the membrane according to the invention, calculated as the ratio of $SC_{Myo}/SC_{Albu}$, accordingly is between 9.4 and 10 or higher.

Another object of the present invention is the use of the membrane in haemodialysis, haemodiafiltration, haemofiltration, and in dialysis and filtration in general, for example for water purification or dehydration.

Other objects, features, advantages and preferred embodiments of the present invention will become apparent from the following detailed description when taken in conjunction with the enclosed scanning micrographs and the appended claims.

Brief Description of the Drawings

Preferred embodiments of the present invention will now be described in more detail. Reference will be made to enclosed drawings.

Figure 1 depicts an electron micrograph of the outer surface of a membrane according to the invention and as prepared according to Example 1. The pores have a diameter of between 1 and 3 $\mu$m. There are about 45000 pores available per mm$^2$. The black arrow indicates 100 $\mu$m.

Figure 2 shows an electron micrograph of a cross-section of the membrane according to the invention and according to Example 1. The 4-layer structure can be seen, including the inner, blood contacting layer, which is a separation layer, the second layer having sponge structure, the third layer in the form of a finger structure and the fourth outer layer with an outer surface as shown in Figure 1.

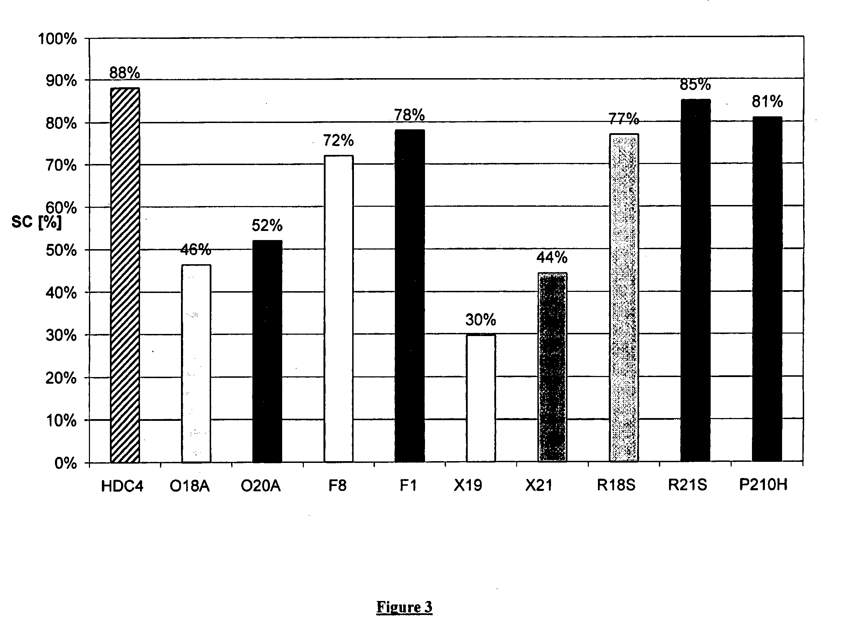

Figure 3 shows the sieving coefficients for the middle molecule myoglobin in % for a membrane according to the invention, depicted as "HDC4" (Example 1) and, for comparison, for additional membranes of the state of the art. All membranes shown are based on polysulfone or polyethersulfone and PVP. As can be seen, the sieving coefficient of the HDC4 membrane is clearly improved and reaches 88% removal.

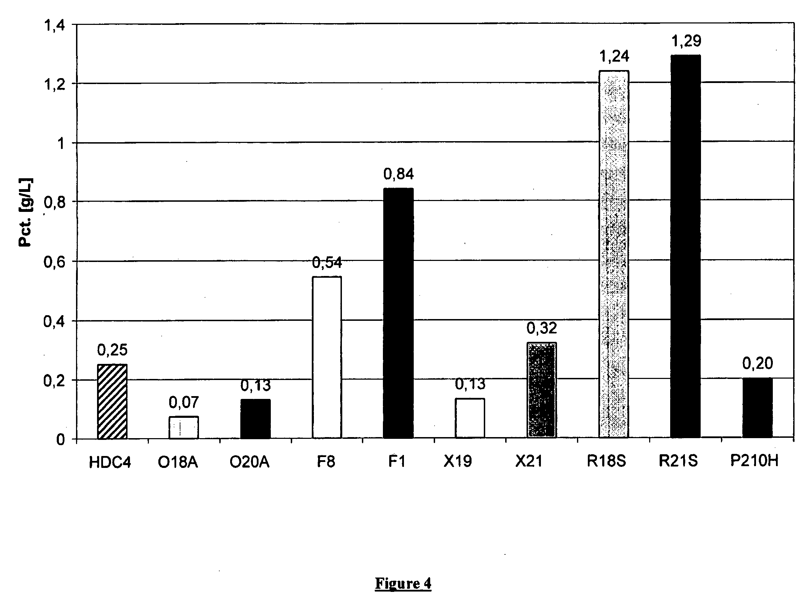

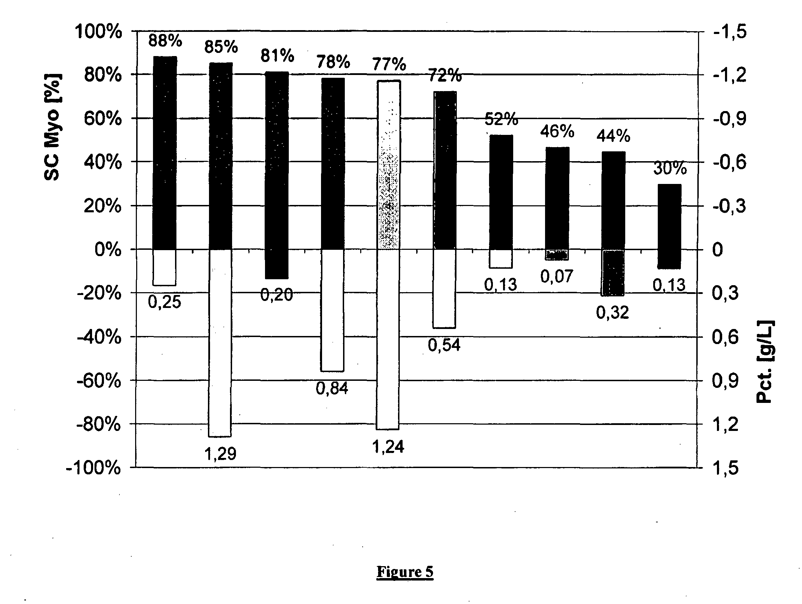

Figure 4 shows the loss of protein, such as albumin, in g/l for a membrane according to the invention, depicted as "HDC4" (Example 1) and, for comparison, for additional membranes of the state of the art. All membranes shown are based on polysulfone or polyethersulfone and PVP. As can be seen, the loss of protein, if not the lowest of all tested membranes, is among the lowest achieved. This is remarkable in connection with the high removal rate for the so-called middle molecules (see, for example, Figure 3 and Example 2), indicating that the membrane of the present invention has a strongly improved ability to select between molecules of medium size and larger proteins which are preferably retained ("selectivity", see Fig. 5).

Figure 5 shows both the sieving coefficient for myoglobin in % and the protein loss in g/l, for comparison. Figure 5 thus summarizes the results as shown in Figures 3 and 4. As can be seen from this Figure, the membranes according to the state of the art which have a low loss of protein suffer from a low removal rate for middle molecules such as myoglobin, whereas those with a good efficacy in removing said middle molecules suffer from a higher loss of protein. The membrane according the present invention is able to combine best performance abilities in both fields.

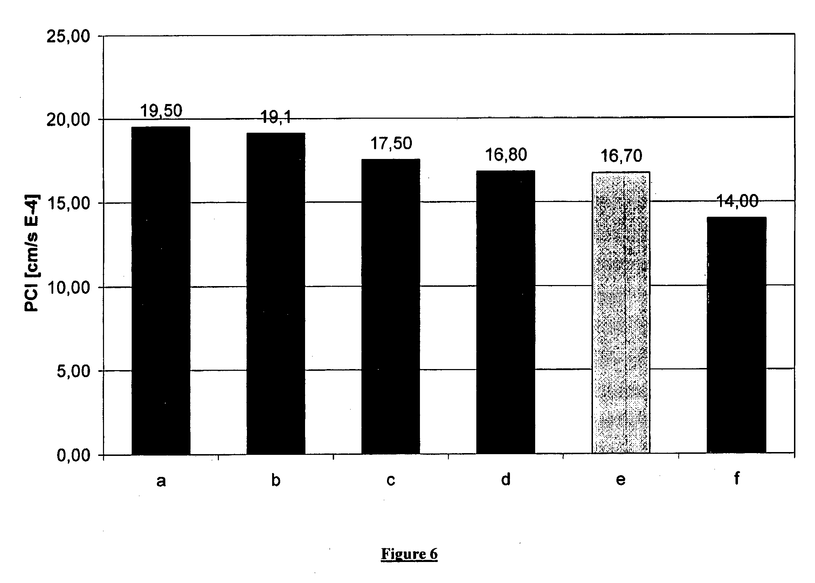

Figure 6 shows the results for the diffusive permeability for chloride in cm/s·$10^{-4}$ for a membrane according to the invention (b) (Example 1) and, for comparison, for additional membranes of the state of the art, (a) and (c)-(f) (Example 5). All membranes shown are based on polysulfone or polyethersulfone and PVP. As can be seen, the membrane according to invention, in spite of its increased sieving coefficient for middle molecules and reduced loss of protein does not suffer from a lower diffusive permeability for small molecules.

Detailed Description of the Invention

The semipermeable hollow membrane according to the invention is based on at least one hydrophobic polymer and on at least one hydrophilic polymer. Said at least one hydrophobic polymer is polyethersulfone.

Said at least one hydrophilic polymer is polyvinylpyrrolidone, wherein the polyvinylpyrrolidone consists of a low molecular weight component having a molecular weight of below 100 kD and a high molecular weight component having a molecular weight of 100 kD or more.

The membrane according to the present invention consists of 80-99% by weight of said hydrophobic polymer, polyethersulfone, and 1-20% by weight of said at least one hydrophilic polymer, polyvinylpyrrolidone (PVP). The PVP consists of a high (≥ 100 kD) and low (< 100 kD) molecular component, wherein the PVP consists of 10-45 weight-% based on the total weight of PVP in the membrane, of a high molecular weight component, and of 55-90 weight-% based on the total weight of PVP in the membrane, of a low molecular weight component.

The spinning solution for preparing a membrane according to the present invention preferably comprises between 12 and 15 weight-% of polyethersulfone or polysulfone as hydrophobic polymer and 5 to 10 weight-% of PVP, wherein said PVP consists of a low and a high molecular PVP component. The total PVP contained in the spinning solution consists of between 22 and 34 weight-% and preferably of between 25 and 30 weight-% of a low molecular weight component and of between 66 and 78 weight-%, preferably of between 70 and 75 weight-% of a high molecular weight component. Examples for high and low molecular weight PVP are, for example, PVP K85/K90 and PVP K30, respectively.

The polymer solution, used to prepare the membrane of the present invention preferably further comprises 66-86 % by weight solvent and 1-5% by weight suitably additives. Suitable additives are, for example, chosen form the group of water, glycerol and/or other alcohols. Water is especially preferred and is present in the spinning solution in an amount of between 1-8% by weight, preferably in an amount of between 2-5% by weight. The solvent used in the process of the present invention preferably is chosen from the group comprising n-methylpyrrolidone (NMP), dimethylacetamide (DMAC), dimethylsulphoxide (DMSO), dimethylformamide (DMF), butyrolactone and mixtures of said solvents. NMP is especially preferred in the context of the present invention. The spinning solution should be homogenously degassed and filtered.

The center fluid or bore liquid which is used for preparing the membrane according to the invention comprises at least one of the above- mentioned solvents and precipitation medium chosen from the group of water, glycerol and other alcohols. Most preferably the center fluid consists of 45-70% by weight precipitation medium, 30-55% by weight of solvent. Preferably, the center fluid consists of 51-57% by weight of water and 43-49% by weight of NMP. Again the center fluid should be degassed and filtered.

The viscosity of the polymer solution should be in the range of between 2500 and 7000 mPa·s, preferably between 3500 and 5500 mPa·s.

In a preferred embodiment the temperature of the spinneret is 30-70°C, preferably 45-55°C, the temperature of the spinning shaft is 25-65°C, preferably 40-50°C. The distance between the opening of the nozzle and the precipitation bath is between 25 and 1500 mm, preferably between 550 and 1100 mm. The precipitation bath has a temperature of 10-40°C, preferably of 15-25°C. The spinning velocity preferably should be in the range of between 25-80 m/min, preferably between 30-60 m/min. The temperature of the humid steam/air mixture is at least 15°C, preferably at least 30°C, and at most 75°C, but is preferably not higher than 60°C. Further, the relative humidity in the humid steam/air mixture is between 60 and 100%.

In another embodiment the humid steam/air mixture comprises a solvent in a content of between 0 and 5% by weight related to the water content. Preferably, the humid steam/air mixture may comprise a solvent in a content of between 0 and 3% by weight related to the water content. The effect of the solvent in the temperature controlled steam atmosphere is to control the speed of precipitation of the fibres. If less solvent is employed, the outer surface will obtain a more dense surface, and if more solvent is used the outer surface will have a more open structure. By controlling the amount of solvent within the temperature controlled steam atmosphere surrounding the precipitating membrane, the amount and size of the pores on the outer surface of the membrane can be modified and controlled.

The membrane according to the present invention will then preferably be washed in water to remove waste components, and then be dried at temperatures between 150-280°C, preferably between 180-260°C. Such drying will provide for an adequate evaporation of water and a defined shrinkage of pores. The final treatment consists of rinsing the membrane in water at a temperature of 50-95°C, preferably 80-90°C and subsequent drying at temperatures of 30-65°C, preferably 55-65°C.

The membrane is preferably steam sterilized at temperatures above 121°C for at least 21 minutes.

In Figure 2, an electron scanning micrograph is shown of the resulting membrane having a four-layer structure. The inner layer of the four-layer structure, i.e. the blood contacting layer and the inner surface of the hollow fiber membrane, is a separation layer in form of a dense rather thin layer having, in a preferred embodiment, a-thickness of less than 1pm and a pore size in the nano-scale range. To achieve high selectivity the pore channels are short, preferably

below 0.1 $\mu$m. The pore channel diameter has a low variation in size. The next layer in the hollow fiber membrane is the second layer having the form of a sponge structure and, in a preferred embodiment of the present invention, a thickness of about 1 to 15 $\mu$m and serving as a support for said first layer. Then, there is the third layer having the form of a finger structure. It provides mechanical stability on the one hand and on the other hand it has a very low resistance of transport of molecules through the membrane due to the high void volume. During the process the voids are filled with water and the water gives a lower resistance for diffusion and convection than a matrix with a sponge- filled structure having a lower void volume. Accordingly, the third layer gives the membrane mechanical stability and has, in a preferred embodiment of the present invention, a thickness of about 20 to 60 $\mu$m. The fourth layer in this preferred embodiment of the present invention is the outer layer. This fourth layer has in a preferred embodiment a thickness of about 1 to 10 $\mu$m.

The membrane according to the invention has a preferred inner diameter of between 180 and 200 $\mu$m. Preferably, the inner diameter should be around 190 $\mu$m. The wall thickness of the hollow fiber should be between 30 and 40 $\mu$m, preferably the wall thickness should be around 35 $\mu$m.

Such spinning and final treatment conditions as described in combination with the specific geometry of the fiber i.e. wall thickness, will provide for the above-disclosed 4-layer asymmetric hollow fiber membrane with improved selectivity and diffusive permeability.

The high performance rates of the membrane according to the present invention allow to devise dialyzers which are significantly reduced in sized without suffering from a reduced performance, e.g. in connection with the clearance rate of small molecules. The reduction is based on the improved performance of the membrane, as well as the reduced wall thickness, length and diameter of the membrane which is needed to arrive at the same or improved results as present day dialyzers. Accordingly, the membrane according to the present invention can be used to design a dialyzer with reduced size, as summarized in Table I. Table I compares the size of dialyzers which are state of the art with a dialyzer prepared on the basis of the membrane according to the invention. Table I gives exemplary data for inner diameter (di), wall thickness (w.th.), effective fiber length ($l_{eff}$), number of fibers per dialyzer (n) and effective surface area ($A_{eff}$). As can be seen, the dialyzer based on the present membrane, depicted as "HDC4", can be reduced, especially with regard to wall thickness and effective surface area compared to membranes which are state of the art, without having to cope with reduced performance efficacy (see Fig. 3 to 6 and Tables II-IV, where the same dialyzers/membranes are being compared).

| Membrane/Dialyzer Type | di [$\mu$m] | w.th. [$\mu$m] | $l_{eff}$[mm] | n | $A_{eff}$ |
|---|---|---|---|---|---|
| HDC4 | 190 | 35 | 236 | 12024 | 1.69 |
| O18 | 190 | 41 | 230 | 12192 | 1.67 |
| O20 | 190 | 41 | 230 | 13974 | 1.92 |
| F8 | 175 | 40 | 226 | 13015 | 1.62 |
| F10 | 1688 | 44 | 226 | 16849 | 2.01 |
| X19 | 195 | 34 | 268 | 11170 | 1.83 |
| X21 | 195 | 34 | 283 | 11955 | 2.07 |
| P21H | 215 | 50 | 260 | 11640 | 2.04 |

The expression "selectivity" as used in the present application refers to the unique ability of the membrane as described here to remove so-called middle sized molecules (500-60000 Dalton), while the loss of valuable proteins having a higher molecular weight, such as, for example, albumin, should be low. The specific selectivity of a membrane can be described by determining the sieving coefficient, in water or plasma, of the middle molecule myoglobin, having a molecular weight of 17053 D, and of the protein albumin, having a molecular weight of 66248 D, i.e. $S_{Myo}/SC_{Albu}$.

The membrane according to the present invention provides for a sieving coefficient for myoglobin as measured in an aqueous solution of between 86% and 90%, preferably about 88%, i.e. 88% of the myoglobin present will be removed from the solution. Measurements are done in PBS-buffer

The expression convective permeability (coefficient) Lp generally refers to the transport flux of the solvent, per unit pressure difference across the membrane. The solute permeability (coefficient), Ls represents the transport flux of the solute per unit concentration difference. The expression "convective permeability" in the context of the present invention generally refers to the Lp of a membrane.

Convective and diffusive permeability are strongly dependent on the wall thickness. Membrane thickness is one important determinant of diffusive transport. Another major determinant of dialyzer membrane diffusive transport is porosity, also known as pore density. Membrane porosity is directly proportional to both the number of pores and the square of the pore radius. Accordingly, the major determinants of flux and diffusive permeability are the number of pores, the square of the pore radius and wall thickness.

The porosity, or per cent void volume, of the membrane according to the invention is, high, i.e. 80% or higher.

The membrane according to the present invention has been optimized with regard to all determinants controlling flux and diffusive permeability, thus resulting in a membrane which reaches values for Lp and $P_{Cl}$, that have not been described before, especially in connection with the exceptionally good selectivity as described before. The convective permeability Lp is in the range of between $56 \cdot 10^{-4}$ and $84 \cdot 10^{-4}$ cm/bar·s, preferably between 70 and $80 \cdot 10^{-4}$ cm/bar·s, which is highest compared to membranes of the prior art (see Table II). Table II compares exemplary values for convective permeability of the membrane according to the invention ("HDC4") and other membranes (dialyzers) of the prior art (see Ex. 4).

**Table II**

| Parameter | Unit | HDC4 | P21H | X | F | O |
|---|---|---|---|---|---|---|
| Convective Permeability Lp | cm/ bar·s | 75 | 62 | 44 | 39 | 25 |

The chloride permeability $P_{Cl}$ is in the range of between $18 \cdot 10^{-4}$ and $21 \cdot 10^{-4}$ cm/s, preferably between $19 \cdot 10^{-4}$ and $20 \cdot 10^{-4}$ cm/s (see Figure 6).

The membrane is further characterized by a high selectivity, i.e. a high removal rate for middle molecules while at the same time the loss of protein of higher molecular weight is minimized. The membrane according to the invention has a sieving coefficient ($SC_{Myo}$) for myoglobin (17053 Dalton) in aqueous solution of between 85 and 90%, and a sieving coefficient ($SC_{Albu}$) for albumin (66248 Dalton) in aqueous solution of 9% or less. The selectivity in aqueous solution of the membrane according to the invention, calculated as the ratio of $SC_{Myo}/SC_{Albu}$, accordingly is between 9.4 and 10 or higher.

Examples

**Example 1**

Preparation of the membrane

A polymer solution is prepared by mixing 14.0 wt.-% of polyethersulfone, 5.0 wt.-% of PVP K30, 2.0 wt.-% of PVP K85/K90, 3 wt.-% of water and 76.0 % of NMP. A mixture of 55 wt.-% water and 45 wt.-% NMP serves as center fluid. The viscosity of the polymer solution, measured at a temperature of 22°C, is 4230 mPa·s.

The center fluid is heated to 55°C and pumped towards a two-component hollow fiber spinneret. The polymer solution is leaving the spinneret through an annular slit with an outer diameter of 0.5 mm and an inner diameter of 0.35 mm. The center fluid is leaving the spinneret in the center of the annular polymer solution tube in order to start the precipitation of the polymer solution from the inside and to determine the inner diameter of the hollow fiber.

At the same time the two components (polymer solution and center fluid) are entering a space separated from the room atmosphere. The space is called spinning shaft. A mixture of steam (100° C) and air (22°C) is injected into the spinning shaft. The temperature in the spinning shaft is adjusted by the ratio of steam and air at about 45°C and a relative humidity of 99,5%. The length of the spinning shaft is 890 mm. By the aid of gravity and a motor-driven roller, the hollow fiber is drawn from top to bottom, from spinneret through the spinning shaft into a water bath in vertical direction. The spinning velocity is 50 m/min. The hollow fiber is subsequently led through a cascade of water bathes and temperatures increasing from 15 to 40°C. The wet hollow fiber membrane leaving the water-rinsing bath is dried in a consecutive online drying step. After a texturizing step, the hollow fiber is collected on a spinning wheel in the shape of a bundle. Alternatively, hand bundles according to Example 7 can be formed.

After introducing the bundle into a dialyser housing, it is potted with polyurethane, ends are cut, on both sides of the dialyser a header is fixed to the housing, the dialyser is rinsed with hot water and dried with air. During this last drying step, an amount of 17 g of residual water per m$^2$ effective membrane area is left on the dialyser. After labelling and packaging, the dialyser is steam-sterilized within the package in an autoclave at 121°C for 25 min.

Scanning micrographs of the outer surface and of the 4-layer structure of the hollow fiber according to Example 1 are shown in Figures 1 and 2. The hollow fiber according to this example had 45500 pores in the range of 1 to 3 $\mu$m per mm2. The inner diameter was adjusted to be 190 $\mu$m and the wall thickness was chosen to be 35 $\mu$m.

**Example 2**

Sieving coefficients for myoglobin and albumin and selectivity of the membrane

The sieving coefficient of a membrane according to Example 1 was determined for myoglobin and albumin in PBS buffer (8 g NaCl, 0.2 g KCl, 1.44 g $Na_2HPO_4$, 0.24 g $KH_2PO_4$ in 800 ml of distilled $H_2O$; pH adjusted to 7.4 with HCl; $H_2O$ added to give 1 liter; sterilized by autoclave) for 25 minutes. The myoglobin concentration in the solution was 100mg/l PBS-buffer. The albumin concentration in the solution was 60 g/l PBS-buffer.

Generally, for all tested membranes (comparative examples with membranes/dialyzers of the state of the art), the following equation for adjusting the QB was used: $\gamma \cdot n \cdot \pi \cdot di^3 \cdot 60/32 + J_v \cdot A \cdot 30$, wherein $\gamma$ is 461 l/s and $J_v$ is $0.0704 \cdot 10^{-4}$ cm/s for all so-called High-Flux membranes as in the present cases, n is the number of fibers in the bundle (12024), di is the inner diameter of the fiber in $\mu$m (190) and A is the membrane surface in $cm^2$ (1.8). For adjusting the UF, the following equation was used: $J_v \cdot A \cdot 60$. The values used for the membrane according to the inventions are exemplarily given in brackets.

15 minutes after the beginning of the test, first samples are taken. One is taken at the blood side (A), one from the retentate (R) and one from the filtrate (F). The sieving coefficient (SC) is determined according to the equation $SC[\%] = 2 \cdot c(F)/[c(A)+c(R)] \cdot 100\%$.

Figure 3 shows the results for the SC of myoglobin for the membrane according to the invention (see Example 1) in column "HDC4". All other columns show results for polysulfone or polyethersulfone and PVP based membranes/dialyzers of the prior art, for comparison.

The sieving coefficient for albumin was also determined. In an aqueous (PBS) solution, the SC for the membrane according to the invention was determined to be 9%, resulting in a selectivity for the membrane of $[S_{Myo}/SC_{Albu}]$ of 9.8 or $[S_{Myo}-SC_{Albu}]$ of 79%, see Table III.

Table III summarizes the results for the SC for myoglobin and albumin for the membrane according to the invention ("HDC4") and for membranes (dialyzers) which are the current state of the art. HDC4 provides for a very good combination of the SC of myoglobin and albumin, i.e. selectivity as described before. The data have been obtained in aqueous solutions. Corresponding results can be obtained when the SC is measured in plasma.

**Table III**

| Parameter | Unit | HD C4 | O180 A | O200 A | F8 | F1 | X19 | X21 | R18 S | R21 S | P210 H |
|---|---|---|---|---|---|---|---|---|---|---|---|
| SC Myo | % | 88 | 46 | 52 | 72 | 78 | 30 | 44 | 77 | 85 | 81 |
| SC Alb | % | 9 | 4 | 3 | 2 | 7 | 2 | 2 | 7 | 8 | 9 |
| $S_{Myo}/SC_{Albu}$ | - | 9.8 | 10.7 | 17.3 | 36.0 | 11.1 | 14.8 | 22.2 | 11.0 | 10.6 | 9.0 |
| $S_{Myo}-SC_{Albu}$ | % | 79 | 42 | 49 | 70 | 71 | 28 | 42 | 70 | 77 | 72 |

**Example 3**

Loss of protein

The analysis of the removal of valuable protein, such as albumin is determined by pumping bovine blood (protein content of 60 g/l) with a defined haematocrit (32%) under defined conditions (QB=400 ml/min; TMP 300 mmWs; 37°C) through a membrane bundle and determining the concentration of the total protein in the feed, in the retentate and in the filtrate. The sampling takes place at the earliest 10 minutes after a constant TMP is adjusted. The test is carried out in the single-pass modus. The bovine blood is heated up by a heat exchanger to 37 °C before entering the fibre bundle. The retentate and the feed samples are centrifuged before the determination of the concentration of the total protein. The determination of the total protein is done photometrical.

Figure 4 shows the results for a membrane prepared according to the invention (see Example 1) and other membranes of the state of the art for comparison. The values given (Pct.) refer to the total loss of protein in g/l. Figure 5 shows the combination of Figures 3 and 4 by showing both the sieving coefficient for myoglobin and the protein loss, thereby demonstrating the specific advantage of the membrane according to the invention.

**Example 4**

Convective Permeability Lp

The convective permeability was determined with a hand bundle as described in Example 7. For determining the Lp of a given hand bundle, said hand bundle is sealed at one end and a defined amount of water passes through the bundle under a certain pressure. This process will take a certain time. Based on said time, the membrane surface area, the pressure used and the volume of the water which has passed the membrane, the Lp can be calculated. The equation used is

$$Lp = \frac{V}{p \times A \times t} = \frac{V}{\pi \times d \times l \times n \times p \times t}$$

wherein Lp is the convective permeability [$\cdot 10^{-4}$ cm/bar·s], V is the water volume [cm$^3$], p is the pressure [bar], t is the time, and A is the effective membrane surface of the bundle with A=$\pi$·d·l·n (see Examples 2 and 5 for the values used here). The pressure used was 400 mmHg. Table IV summarizes the results for the Lp for the membrane according to the invention (Example 1) and other polysulfone/polyethersulfone and PVP based membranes (dialyzers) according to the state of the art, for comparison. As can be seen there, the membrane according to the present invention ("HDC4") achieves a significantly higher Lp than membranes of the state of the art.

| Para-meter | Unit | HDC4 | O180 A | O200 A | F8 | F1 | X19 | X21 | R18 S | R21 S | P210 H |
|---|---|---|---|---|---|---|---|---|---|---|---|
| Convec-tive perme-ability | [cm/(bar·s)] $\cdot 10^{-4}$ | 75 | 24 | 27 | 40 | 38 | 41 | 46 | 45 | 52 | 62 |

**Table IV**

**Example 5**

Diffusive Permeability P$_{Cl}$

The diffusive permeability was determined with a hand bundle as described in Example 7. First, the diffusion experiment is performed, followed by chloride analysis with AgNO$_3$. The AgNO$_3$ solution used had a concentration of 0.100 mol/l. The diffusive permeability of chloride is calculated according to the following equation:

$$P_{Cl} = \frac{Q_B}{60 \times A} \times \ln \frac{c_A - c_D}{c_R}$$

wherein $c_A$ is the starting concentration of NaCl, $c_D$ is the concentration in the dialysate, $c_R$ is the concentration in the retentate, $Q_B$ is the blood flow [ml/min], A is the effective membrane surface of the hand bundle with A=$\pi$·d·l·n (see also Example 2; l is the effective fiber length, 236 mm for the membrane according to the invention; d is 190 $\mu$m, n is 12024). Measurements were done at 37°C and $Q_B$ 4ml/min. The $Q_D$ was 3000 ml/min. The first samples were taken 10 minutes after the start. Samples were collected for three minutes, and the chloride ion concentration is determined for each sample taken with a TTT 85 TITRATOR. Figure 6 shows the loss of protein for a membrane according to the invention (Example 1) and other membranes (dialyzers) of the state of the art in g/l. Again, it is important to compare the results for this parameter with the efficiency of middle molecule removal for assessing the progress which has been achieved with the present membrane.

**Example 6**

Urea clearance

The method for determining the clearance for urea was EN1283. $Q_B$=400 ml/min, $Q_D$=700 ml/min and $U_F$=0 ml/min. Again, the membrane according to the present invention (see Example 1) was compared to membranes (dialyzers) of the state of the art in order to demonstrate that the achievement with regard to permeability and selectivity has no negative influence on the efficiency of the membrane as to the removal of small molecules. As can be seen from Table V, the clearance for urea, as an example for the clearance of small molecules, is among the highest which are currently achieved in dialyzer technology.

**Table V**

| Parameter | Unit | HD C4 | O180 A | O200 A | F8 | F1 | X19 | X21 | R18 S | R21 S | P210 H |
|---|---|---|---|---|---|---|---|---|---|---|---|
| Clearance Urea | ml/min | 366 | 335 | 354 | 347 | 366 | 356 | 365 | 359 | 370 | 360 |

**Example 7**

Preparation of hand bundles

The preparation of membrane bundles after the spinning process is necessary to prepare the fibre bundle in an adequate way for the performance tests. The first process step is to fix the fibres near their ends by a filament. The fibre bundle is transferred into a sterilization tube and then sterilized. After the sterilization, the fibre bundle is cut to a defined length of 23 cm. The next process step consists of closing the ends of the fibres. An optical control ensures that all fibres are well closed. Then, the ends of the fibre bundle are transferred into a potting cap. The potting cap is fixed mechanically, and a potting tube is put over the potting caps. Afterwards, the potting is done with polyurethane. After the potting, it has to be ensured that the polyurethane can harden for at least one day. In the next process step, the potted membrane bundle is cut to a defined length. The last process step consists of an optic control of the fibre bundle. During this process step, the quality of the cut (is the cut smooth or are there any damages of the knife) and the quality of the potting (is the number of open fibres of the spinning process reduced by fibres that are potted or are there any visible voids where the there is no polyurethane) are controlled. After the optical control, the membrane bundles are stored dry before they are used for the different performance tests.

**Claims**

1. A semipermeable asymmetric hollow fiber membrane, the membrane material consisting of 80-99% by weight of a polyethersulfone and 1-20% by weight of a polyvinylpyrrolidone, wherein the polyvinylpyrrolidone consists of a low molecular weight component having a molecular weight of below 100 kD and a high molecular weight component having a molecular weight of 100 kD or more, **characterized in that** the polyvinylpyrrolidone consists of 10-45 weight-% based on the total weight of PVP in the membrane, of the high molecular weight component and 55-90 weight.-%, based on the total weight of PVP in the membrane, of the low molecular weight component, wherein the membrane has a four-layer structure comprising a first inner separation layer in form of a dense rather thin layer having a thickness of less than 1 $\mu$m and a pore size in the nano-scale range, a second layer in the form of a sponge structure, a third layer in form of a finger structure, and a fourth outer layer having an outer surface having pores in the range of 0.5-3 $\mu$m and having a number of said pores in the range of 10,000 to 150,000 pores per mm$^2$, and wherein the membrane has a sieving coefficient for myoglobin in aqueous solution of between 86% and 90%, a convective permeability Lp of water at 37°C of between 56x10$^{-4}$ and 84x10$^{-4}$ cm/bar x s, a diffusive permeability $P_{Cl}$ of chloride at 37°C of between 18x10$^{-4}$ and 21x10$^{-4}$ cm/sec; and is **characterized by** a selectivity $SC_{Myo}/SC_{Albu}$ of 9.4 or higher.

2. Use of a membrane according to claim 1 in filtration and dialysis applications.

**Patentansprüche**

1. Semipermeable asymmetrische Hohlfasermembran, deren Membranmaterial aus 80-99 Gew.% eines Polyethersulfons und 1-20 Gew.% eines Polyvinylpyrrolidons besteht, wobei das Polyvinylpyrrolidon aus einer Komponente mit niedrigem Molekulargewicht mit einem Molekulargewicht von weniger als 100 kD und einer Komponente mit hohem Molekulargewicht mit einem Molekulargewicht von 100 kD oder mehr besteht, **gekennzeichnet dadurch, dass** das Polyvinylpyrrolidon aus 10-45 Gew.%, bezogen auf das Gesamtgewicht von PVP in der Membran, der Komponente mit hohem Molekulargewicht und 55-90 Gew.%, bezogen auf das Gesamtgewicht von PVP in der Membran, der Komponente mit niedrigem Molekulargewicht besteht, wobei die Membran eine vierschichtige Struktur aufweist umfassend eine erste innere Trennschicht in Form einer dichten eher dünnen Schicht mit einer Dicke von weniger als 1 $\mu$m und einer Porengröße im Nanometerbereich, eine zweite Schicht in Form einer Schwammstruktur, eine dritte Schicht in Form einer Fingerstruktur, und eine vierte äußere Schicht, die eine äußere Oberfläche mit Poren im Größenbereich von 0.5-3 $\mu$m und einer Porenanzahl im Bereich von 10,000 to 150,000 Poren per mm$^2$ aufweist, and wobei die Membrane einen Siebkoeffizienten für Myoglobin in wässriger Lösung von zwischen 86% und 90%, eine konvektive Permeabilität Lp für Wasser bei 37°C von zwischen 56x10$^{-4}$ und 84x10$^{-4}$ cm/bar x s, eine diffusive Permeabilität $P_{Cl}$ für Chlorid bei 37°C von zwischen 18x10$^{-4}$ und 21x10$^{-4}$ cm/sec aufweist; und **gekennzeichnet ist durch** eine Selektivität $SC_{Myo}/SC_{Albu}$ von 9.4 oder höher.

2. Verwendung einer Membran gemäß Anspruch 1 in Filtrations- und Dialyseanwendungen.

**Revendications**

1. Membrane en fibres creuses asymétrique, dans laquelle le matériau de la membrane est constitué de 80-99 % en poids d'un polyéther sulfone et 1-20 % en poids d'un polyvinyle pyrrolidone, et ledit polyvinyle pyrrolidone consiste en un composant de faible poids moléculaire ayant un poids moléculaire inférieur à 100 kD, et un composant de haut poids moléculaire ayant un poids moléculaire de 100 kD ou plus, **caractérisé en ce que** ledit polyvinyle pyrrolidone est constitué de 10 à 45% en poids par rapport au poids total de PVP dans la membrane, dudit composant ayant un poids moléculaire élevé, et de 55-90% en poids par rapport au poids total de PVP dans la membrane, dudit composant de faible poids moléculaire, laquelle membrane présente une structure à quatre couches comprenant une première couche de séparation intérieure sous la forme d'une couche dense et plutôt mince ayant une épaisseur inférieure à 1 $\mu$m et une taille des pores à l'échelle nanométrique, une seconde couche sous la forme d'une structure en éponge, une troisième couche sous la forme d'une structure digitale, et une quatrième couche extérieure ayant une surface extérieure avec des pores dans la plage de 0,5 à 3 $\mu$m, et dont le nombre desdits pores sur la surface extérieure est dans la plage de 10 000 à 150 000 pores par mm$^2$, et laquelle membrane présente un coefficient de criblage pour le myoglobine en solution aqueuse de 86% à 90%, une perméabilité par convection Lp à l'eau à 37°C de 56x10$^{-4}$ à 84x10$^{-4}$ cm/bar x s, une perméabilité par diffusion $P_{Cl}$ au chlorure à 37°C de 18x10$^{-4}$ à 21x10$^{-4}$ cm/sec; et laquelle membrane est **caractérisée par** une sélectivité $SC_{Myo}/SC_{Albu}$ de 9.4 ou plus.

2. Utilisation d'une membrane selon la revendication 1 pour applications de filtration et de la dialyse.

**Figure 1**

**Figure 2**

Figure 3

**Figure 4**

**Figure 5**

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0568045 A1
- EP 0168783 A1
- EP 0082433 A2
- WO 2004056459 A1
- EP 0750936 A1
- WO 8600028 A1
- EP 0305787 A
- WO 2006106133 A1
- US 6355730 B1
- WO 2008003608 A

**Non-patent literature cited in the description**

- **WIENK et al.** A new spinning technique for hollow fiber ultrafiltration membranes. *Journal of Membrane Science,* 1998, vol. 78, 93-100
- **E. KLEIN ; F. HOLLAND ; A. LEBEOUF ; A. DONNAUD ; J. K. SMITH.** Transport and Mechanical Properties of Hemodialysis Hollow Fibers. *Journal of Membrane Science,* 1976, vol. 1, 371-396